# EUROPEAN PATENT APPLICATION

(11) **EP 0 529 089 A1**
(43) Date of publication of application: **03.03.1993**
(21) Application number: 92905841.0
(22) Date of filing: 28.02.1992
(51) Int. Cl.: C12M 1/06, C12M 3/00

(54) **CELL CULTURE TANK**

(30) Priority: 28.02.1991 JP 58094/91; 12.06.1991 JP 167620/91
(71) Applicant: SNOW BRAND MILK PRODUCTS CO., LTD., Sapporo-shi, Hokkaido 065 (JP)
(72) Inventor: NAKAKARUMAI, Tadashi, 405, Ishibashi, Tochigi-ken 329-05 (JP); MANO, Takashi, City-heights Kishi B-101, Tochigi-ken 328 (JP); KAWAHARA, Hiroyuki, 773-3, Ishibashi, Tochigi-ken 329-05 (JP); MATSUDA, Yoshiaki, 203-46, Ohtsutsumi Souwa-machi, Ibaragi-ken 306 (JP); MITSUDA, Shinjiro, 3-3-8, Sakuradai, Saitama-ken 349-01 (JP); KUMAZAWA, Eitaro, Ittoku Haitsu 2-612, Tochigi-ken 323 (JP); BABA, Atsushi, Kurai Mansion 2-A, Tochigi-ken 329-05 (JP)
(74) Representative: Rackham, Stephen Neil
(86) International application number: JP9200222
(87) International publication number: WO9215668

(57) **Abstract**

A cell culture tank (A) having in the cell culture zone (A') thereof a partial zone (B) partitioned with a filter (8) permitting permeation of a culture medium and protein produced by cells under culture but intercepting permeation of cell or cohesive carrier of cells. A jet nozzle (6) of a conduit for feeding oxygen or air is provided on the bottom of the zone (B), stirring vanes (3) rotating at high speed are provided immediately above said jet nozzle, and a space corresponding to 30 to 50 % of the height of the filter (8) is exposed to a gaseous phase part in the cell culture tank. A conduit nozzle (7) for recovering the culture medium is provided just below the medium level in the zone (B). Another stirring vane (1) rotating at low speed is provided in the culture zone (A'). By providing an exhaust pipe (20) and a sterile air ventilation pipe (21) to the gaseous phase part of the culture zone (A') as well as valves to the conduit pipe of jet nozzle (6), exhaust pipe (20), and sterile ventilation pipe (21), respectively, the level of the medium in the zone (B) having been lowered by bubbles developing in the zone (B) is raised and said bubbles are squeezed out to the culture tank (A) through the filter, whereby the bubbles are broken. When an opening is provided in the upper part of the zone (B) and a bubble breaking vane rotating at high speed is provided above the level of medium in said zone (B), bubbles having developed in said zone can be broken.

## Description

### (Technical Field)

The present invention relates to a cell culture tank and more particularly to such tank adapted for so-called high density cell culture having an improved arrangement allowing oxygen of high concentration to be supplied into culture broth while foam caused by oxygen supply as well as by stirring is effectively put out and a quantity of the culture broth is continuously collected.

### (Background)

The term "high density cell culture" generally means culture of 10⁶ to 10⁸ cells per 1 ml of culture broth. Culture of cells, particularly of animal cells at such high density requires supply of nutritive ingredients necessary for growth of the cells, removal of substances as well as waste matter produced as a result of the growth, and rapid supply of oxygen consumed during the high density cell culture process. While said supply of nutritive ingredients and said removal of the waste matter can be easily achieved by continuous supply of fresh culture medium and collection of culture broth, it has been very difficult for the prior art to supply the culture broth with oxygen of high concentration necessary for the high density cell culture.

Conventionally, the supply of oxygen has been carried out by direct aeration of the culture tank with oxygen or air. However, such method has not been able to achieve a sufficiently high dissolution rate of oxygen into the culture broth to supply the culture broth with desired amount of oxygen and an attempt to increase the amount of oxygen to be supplied has disadvantageously been accompanied with undesirable foaming of the culture broth which has been correspondingly increased.

To solve this problem, it has been proposed to improve the dissolution rate by use of stirring. However, this measure has promoted said undesirable foaming and raised another problem of a shearing force which is apt to injure the cells. Particularly in culture of animal cells, such injury of the cells due to the shearing force has been serious.

In view of this situation, there have been proposed various improvements, e.g., an apparatus having a separate zone exclusively used for aeration and utilizing a reversibly rotatable vane wheel as disclosed by Japanese Patent Application Disclosure Gazette No. 1988-177780; an apparatus including semi-permeable membrane through which the oxygen supply occurs as disclosed by Japanese Patent Application Disclosure Gazette No. 1985-234580; and an apparatus utilizing hollow fibers as disclosed by Japanese Patent Application Disclosure Gazette No. 1987-500357. In addition, Japanese Patent Application Disclosure Gazette No. 1988-14687 discloses a culture tank externally equipped with a device used to accelerate dissolution of oxygen into the culture broth and Japanese Patent Application Disclosure Gazette No. 1986-257181 discloses a cultivating method utilizing hollow fibers by which gas exchange and waste matter exchange simultaneously occur.

However, none of these well known arrangements has been successfully used as the high density cultivating apparatus for animal cells particularly because of problems remaining unsolved such as oxygen supply shortage and inconvenient separation between cells and culture broth.

The inventors have proposed an apparatus suitable for the high density cell culture as shown by Fig. 7. Details of this apparatus is described in Japanese Patent Application Disclosure Gazette No. 1989-174376. This apparatus generally comprises, in addition to stirring vanes 12 provided within a culture tank 14, stirring vanes 13 exclusively used for dissolution of oxygen so that both oxygen supply and perfusion culture can be much more efficiently carried out than with the conventional apparatuses. However, said stirring vanes 13 for dissolution of oxygen is directly coupled to a rotary shaft 11 of said stirring vanes 12 provided with the culture tank 14 and rotation of the stirring vanes 13 at high r.p.m. would cause unacceptably significant foaming within the culture tank 14. Thus, the r.p.m. of the stirring vanes 13 is limited, resulting in a reduced efficiency of oxygen dissolution and a shortage of oxygen supply into the culture broth. Furthermore, this apparatus has a structural problem that the culture broth may be foamed as the broth is stirred.

Accordingly, it is an object of the present invention to provide a cell culture tank provided, as a part thereof, with a zone isolated by a filter which is permeable for the culture broth and protein produced by the cells being cultivated but impermeable for cell adhesion carriers, said zone allowing oxygen to be dissolved at a high concentration but maintaining a defoaming effect so that only the culture broth can be collected.

### (Disclosure of the Invention)

To achieve the object set forth above, the present invention generally resides in a cell culture tank (A) comprising a filter 8 being permeable for culture broth as well as protein produced by cells being cultivated but impermeable for the cells or cell adhesion carriers and provided within the cell culture tank (A) so as to isolate a zone (B) from a cultivating zone (A') of the cell culture tank (A), a conduit provided at a bottom of the zone (B) for supply of oxygen or air, stirring vanes of high r.p.m. provided immediately above an injection nozzle associated with said conduit, 30 to 50 % of the filter's height being exposed to a gas phase area within the cell culture tank, and a conduit/nozzle assembly provided immediately below a fluid level within the zone (B) for collection of culture broth. A top of the zone (B) is opened to the gas phase and there may be provided defoaming vanes of high r.p.m. above the fluid level within the zone (B).

It is also possible without departure from the scope of the invention to provide a germ-free air supply pipe in order to raise the broth level within the stirring zone (B) which has been lowered due to the foam generated within said zone (B) and thereby to expel said foam within said zone (B) out through the filter into the culture tank for effective defoaming.

With the suspension culture tank of well known art, the present invention makes it possible to supply said tank with oxygen of a sufficiently high concentration to meet the requirement for the high density cell culture and effectively overcomes the conventional problems of foaming and shearing force as have been described above. Additionally, the invention allows an excellent defoaming effect to be maintained even if oxygen supply is continuously performed and therefore the culture broth can be continuously supplied and collected. In this manner, the high density cell culture can be continued for a long period.

Summary of the invention will be described more in detail. The apparatus of this invention is suitable for culture of anchorage-independent cells or cell culture utilizing microcarriers. While the tank itself may be any type so far as it is suitable for the normal suspension culture, the culture tank of low r.p.m. stirring type is preferably. There is provided one or more zones (B) isolated by respective filters each adapted to be impermeable for cells or cell adhesion carriers. For convenience of explanation, said zones (B) will be referred to as gas/liquid mixing chambers (B). For the anchorage-independent cells, it is preferred to use ceramic filter or the like and, for the microcarriers, stainless steel filter of 50 to 150 meshes. It should be understood, however, that the filters made of the other materials may be used. The filter may comprise a plurality of laminated filters having different pore sizes or mesh sizes or pore sizes, or the filter may be vibrated or rotated in order to prevent the filter from being plugged up with the cells sticking thereto as the culture broth is perfused.

The number of the gas/liquid mixing chambers (B) may be increased as the cell density increases to accelerate the oxygen supply into the culture broth at a correspondingly higher concentration. It is important for rapid permeation and defoaming of the culture broth to expose 30 to 50 % of the gas/liquid mixing chamber's height to a gas phase area of the culture tank and, so far as the defoaming effect is concerned, a portion of the filter associated with such exposed height may be of a larger mesh sizes, or pore sizes if desired.

The gas/liquid mixing chamber is provided at a bottom thereof with a conduit for supply of oxygen or air and there is provided a stirring vane of 100 to 500 r.p.m. above a nozzle of said conduit so as to accelerate dissolution of oxygen into the culture broth under a stirring effect of such high r.p.m.

A culture broth collector conduit is provided below the fluid level within the gas/liquid mixing may be selectively sealed or opened and, in the latter case, a defoaming vane may be located above the fluid level to improve a defoaming efficiency. When the top of the gas/liquid chamber is sealed, this is for the purpose of preventing the foam from flying off externally of the apparatus.

### (Brief Description of the Drawing)

Fig. 1 is an axially sectional view of a cultivating apparatus constructed in accordance with the present invention;
Fig. 2 is a plan view of the cultivating apparatus illustrated in Fig. 1, showing only a half thereof;
Fig. 3 is a fragmentary sectional view of a zone (B) isolated by a filter within the cultivating apparatus wherein a top of said zone (B) is sealed;
Fig. 4 is a view similar to Fig. 3 but showing an alternative arrangement of the zone (B) wherein a top of the zone (B) is opened and there is provided a defoaming vane immediately above a fluid level;
Fig. 5 is a sectional view of a cultivating apparatus having the same zone (B) as in the apparatus of Fig. 1 and additionally having a pressure regulating function;
Fig. 6 is a graphic diagram showing a value of EPO activity obtained in EXAMPLE 2 utilizing the EIA method wherein ordinate indicates a relative EPO activity and abscissa indicates a period of cultivation (days); and

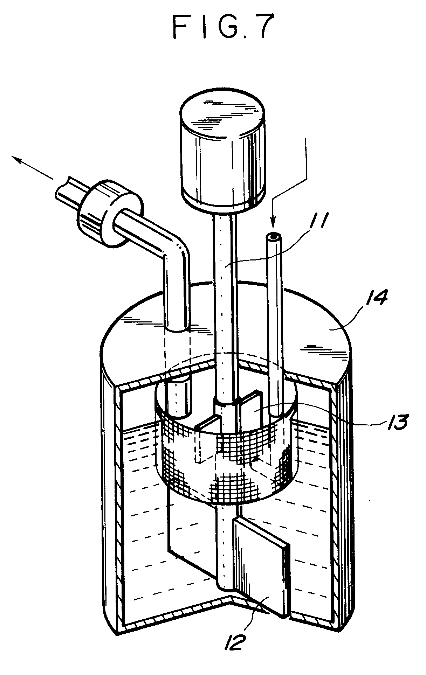

Fig. 7 is a perspective view illustrating, as partially broken away, the rotary filter device of prior art disclosed by Japanese Patent Application Disclosure Gazette No. 1989-174376.

### (Best Mode for Carrying out the Invention)

These and other aspects of the invention will be more fully understood by referring to the following detailed description made in reference with the attached drawings and experimental EXAMPLES.

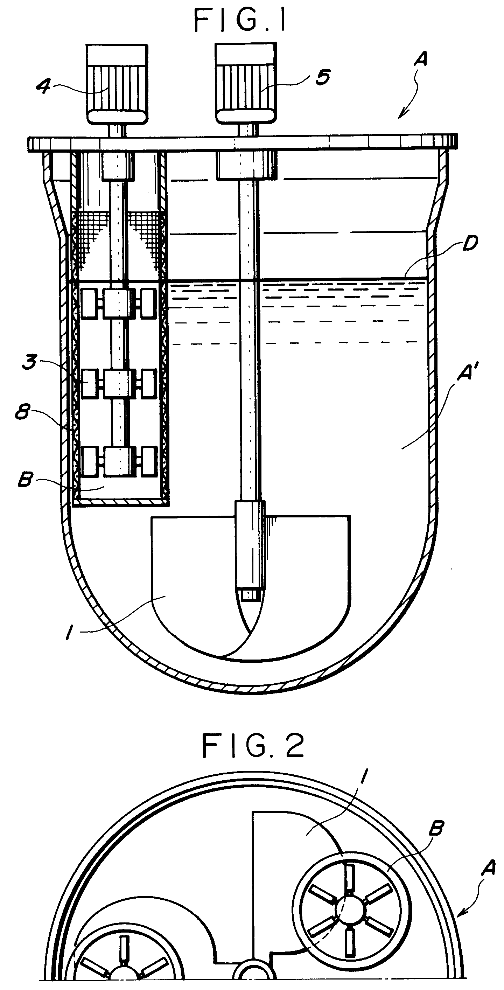

Fig. 1 illustrates a cell culture tank of the invention comprises a cell culture tank proper (A) containing therein culture broth and one or more zones (B) isolated by respective filters 8 within the culture broth. It should be understood here that a cultivating zone within the cell culture tank (A) except the zones (B) will be designated by (A').

The cell culture tank (A) and a main stirring vane (1) have their configuration adapted for suspension culture or microcarrier culture. An electric motor 5 has an ability to rotate the stirring vane 1 at a relatively low velocity, i.e., at a velocity in order of 50 r.p.m.

Each of said zones (B) isolated by the associated filter 8 is stirred by a stirring vane 3 exclusively used for gas/liquid mixing which is, in turn, rotated at a relatively high velocity, i.e., at a velocity in order of 300 r.p.m. by an associated electric motor 4.

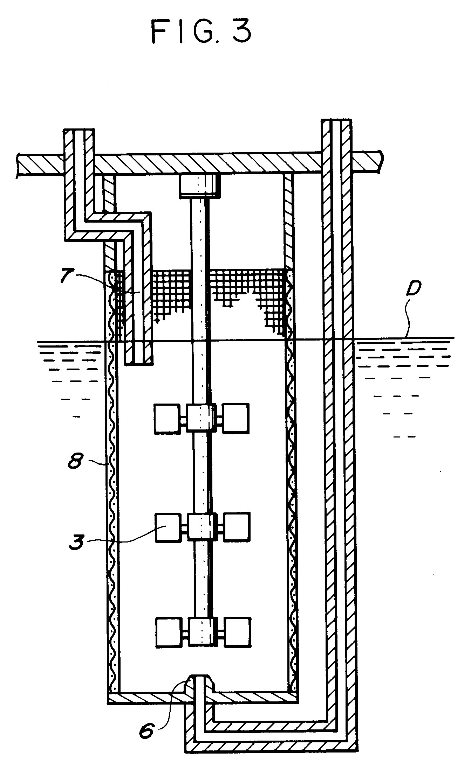

Referring to Fig. 3, structural details of the zone (B) isolated by the filter 8 as shown also in Fig. 1 is provided at a bottom thereof with a nozzle 6 for 7introduction of oxygen or air, and oxygen, for example, discharged through this nozzle 6 is stirred by the stirring vane 3 and dissolved in the culture broth. Foam generated as oxygen or air is introduced into the zone (B) and subjected to stirring is expelled out through meshes of the filter 8 which are exposed above a fluid level (D) of the culture tank over the fluid level of the cultivating zone (A') and defoamed. It has been found that, for the microcarrier culture, the filter 8 of 50 to 150 meshes is free from undesirable plugging and supports a smooth exchange of culture medium. Preferable materials from which the filter 8 is made should be those which are innoxious for the cells and nonadsorptive for gas, such as mono- or multilayered polyester, glass, sintered metal, ceramics and stainless steel. However, any other materials may be used without departure from the scope of the invention.

Immediately below the level of the culture broth, there is provided a culture broth collector conduit 7 having an opening so that a quantity of the culture broth containing neither the cells nor the microcarriers may be collected.

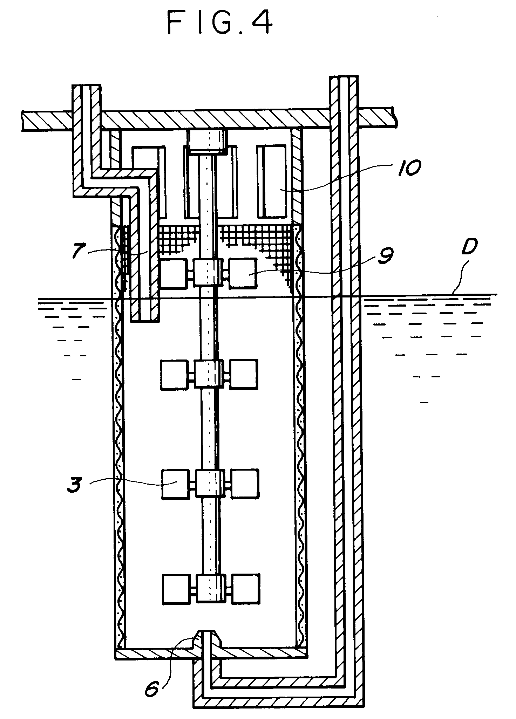

Fig. 4 shows an alternative embodiment being different from the embodiment of Fig. 3, in which a defoaming function is effectuated by a vane 9 provided immediately above the level (D) of the culture broth coaxially with the stirring vane 3.

The vane 9 may be of any configuration so long as it optimally functions for particular properties of the foam generated. The zone (B) is provided at its upper portion with windows 10 opening to the gas phase area of the culture tank so that a pressure change due to the defoaming effect and rotation of the defoaming vane 9 may be kept uniform.

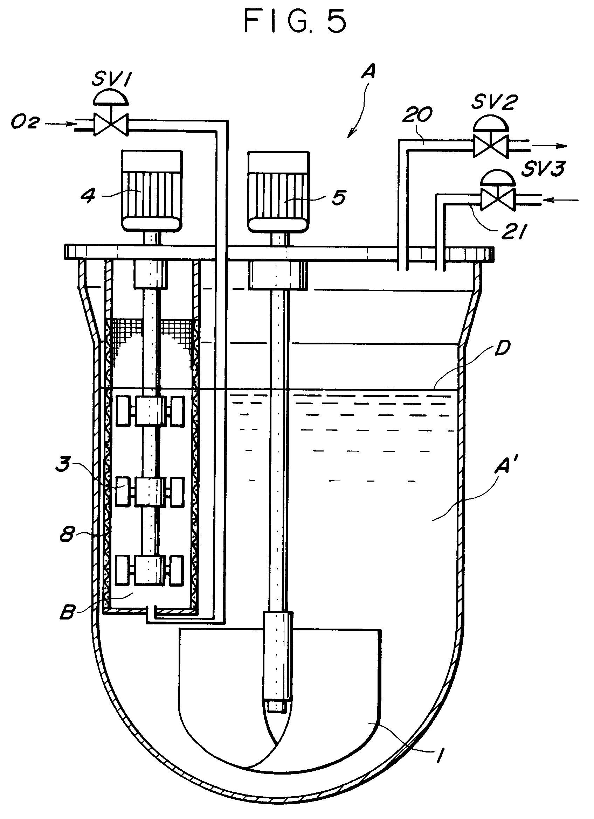

Fig. 5 shows an embodiment being different from the embodiment of Fig. 3 in that the culture tank has, in addition of the same zone (B) as that illustrated by Fig. 3, an exhaust valve SV2 and a pressurized valve SV3 for regulation of a pressure within the culture tank. In the same manner as has already been mentioned with respect to the embodiment of Fig. 3, the zone (B) is subjected to stirring at a high r.p.m. for gas/liquid mixing and any amount of foam thereby generated is effectively defoamed as the foam moves through the filter 8 into the culture tank.

A differential pressure may be established between the zone (A') and the zone (B) of the culture tank to enhance the defoaming effect.

This may be achieved by controllably opening and closing valves SV1, SV2, SV3 shown by Fig. 5 for pressure regulation. SV1, SV2 and SV3 designate the oxygen supply valve, the exhaust valve and the pressurized valve, respectively. The oxygen supply valve SV1 is located in a conduit leading to the nozzle 6, the exhaust valve SV2 is located in an exhaust conduit 20 and the pressurized valve SV3 is located in a pressurized conduit 21 for introducing germ-free air.

Opening and closing of these valves SV1, SV2, SV3 cooperatively occur. More specifically, the valve SV2 is opened and the valve SV3 is closed as the valve SV1 is opened. The valve SV2 is closed and the valve SV3 is opened as the valve SV1 is closed. Opening and closing of these valves in such manner allow the pressure within the tank to be controllably varied.

As has already been described, oxygen supply and high r.p.m. stirring inevitably cause undesirable foam to be generated within the zone (B). Such foam otherwise would depress the fluid level within the zone (B) relative to the fluid level within the zone (A') and progressively swell within the zone (B). However, the valves SV1, SV2 are closed while the valve SV3 is opened, thereby germ-free air is introduced into the culture tank and a pressure within the culture tank correspondingly rises so as to expel a portion of the culture broth from the zone (A') into the zone (B) and thereby to restore the initial fluid level within said zone (B). Thus, the foam generated within the zone (B) is expelled through the filter 8 into the culture tank as the fluid level rises, and this passage through the filter 8 effectuates the desired defoaming.

The stirring vane 3 for the zone (B) operates in association with the above-mentioned opening and closing of the valves. Specifically, the vane 3 begins to be rotated as the valve SV1 is opened and stopped as said valve SV1 is closed. Durations for which the valve SV1 is opened and closed may be adjusted to 3 to 5 seconds and 8 to 13 seconds, respectively, to enhance both the defoaming effect and the oxygen dissolution efficiency. Duration for which the valve is opened is preferably adjusted so that the pressure within the culture tank may be increased by 0.2 to 0.4 kg/cm² as a result of germ-free air supply through this valve SV3.

It should be understood that the above-mentioned adjustment is effective only when the zone (B) is in a substantially sealed condition and the arrangement as shown by Fig. 4 in which the zone (B) has the windows 10 is not suitable for such adjustment since the zones (A') and (B) have a same internal pressure.

As will be apparent from the foregoing description, operation of the invention essentially comprises of introducing oxygen or air into the zone (B) isolated by the filter through the conduit and dissoluting oxygen in the culture broth under the effect of stirring at a high r.p.m.

Consequently, the culture broth contains a sufficient amount of oxygen to support growth of the cells and moves through the filter into the zone (A') of the culture tank. The filter is exposed to the gas phase over a sufficiently large area of the filter to promote a fluid flow between the gas/liquid mixing chamber or the zone (B) and the zone (A') and thereby to effectuate rapid defoaming.

Such defoaming effect is further improved by the defoaming vane constructed and located according to the invention.

The stirring at high r.p.m. is employed by the invention only within the zone containing no cell and therefore the problem none of the conventional apparatuses has been able to overcome, i.e., cell destruction due to a shearing force developed by said stirring at high r.p.m. is effectively avoided. Furthermore, the present invention allows a quantity of the cell-free culture broth to be continuously collected through the culture broth collector conduit.

Additionally, the present invention provides the improved arrangement such that germ-free air is supplied into the culture tank through the germ-free pressurized vent conduit which opens to the gas phase area so as to pressurize the interior of the culture tank and thereby to raise the fluid level within the gas/liquid mixing chamber, allowing the foam generated within the gas/liquid mixing chamber to be expelled out through the filter into the culture tank and thus allowing the desirable defoaming effect to be obtained.

Using the apparatus of the invention as has been described hereinabove, a series of experiments were conducted in comparison with the cultivating methods of prior art. EXAMPLES of these experiments will be described below:

### EXPERIMENT 1

The rate of oxygen supply into the culture broth was determined using the culture tank as has been described in reference with Fig. 3 and simultaneously such values obtained by the conventional methods for oxygen supply were also determined as the controls, i.e., (b) the oxygen supplying method utilizing the silicon tube, (c) the oxygen supplying method utilizing the direct aeration and (d) the oxygen supplying method utilizing the apparatus disclosed in Japanese Patent Application Disclosure Gazette No. 1989-174376 (referred to hereinafter as the rotary filter method). The result will be shown in Table 1.

**Table 1**

| O² supply method | | O² supply rate mmol/h | Cell damage | Foaming |
|---|---|---|---|---|
| a)Invention | rpm | | | |
| | 200 | 61 | ⃝ | ⃝ |
| | 300 | 111 | ⃝ | ⃝ |
| | 400 | 257 | ⃝ | ⃝ |
| b)Silicon tube method | | 58 | ⃝ | ⃝ |
| c)Direct aeration method | | 16 | X | X |
| d)Rotary filter method | | 10 | ⃝ | X |
| ⃝ negligible (result was acceptable) X noticeable (result was unacceptable) | | | | |

These methods were carried out under the following conditions, respectively:
(a) The method of the invention -
   Filter...comprising three layers having pore sizes of 150, 100, 60 meshes, respectively.
   Rotary vane provided within the zone (B) surrounded by the filter...
   three disc turbines each having a diameter of 70 mm.
   Rate of aeration with pure oxygen...
   1 l/min.
(b) Silicon Tube method -
   Outer diameter of silicon tube...
   3 mm.
   Length of silicon tube...
   60 m.
(c) Direct aeration method -
   Rate of aeration...
   1 l/min.
(d) Rotary filter method -
   r.p.m. of filter...
   80 r.p.m.
   Rate of aeration...
   1 l/min.

The amount of dissolved oxygen varying as a time elapses was determined and thereby the oxygen supply rate (mmol/h) was calculated. Foaming in the culture broth containing 2 % serum was also observed and the result shown in Table 1 was obtained.

The result indicated that the apparatus of the invention exhibits a remarkably high rate of oxygen supply and makes the high density culture practically feasible.

### (EXAMPLE 2)

Cell culture was conducted continuously for a long period using the culture tank shown by Fig. 5.

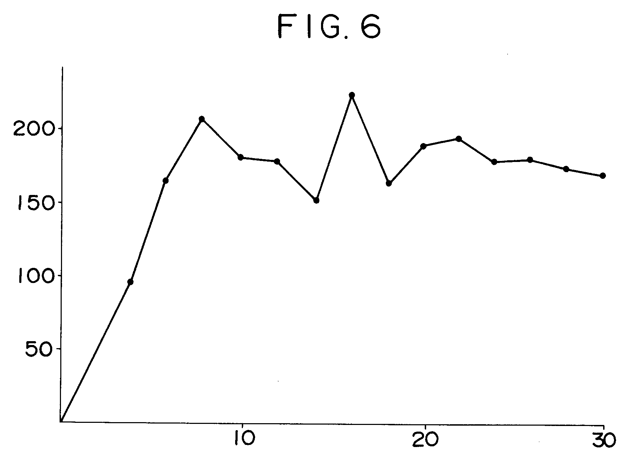

To observe progress of cell culture and substance production, the gene recombinational BHK cells which produce human erythropoietin (EPO) (See Japanese Patent Application Disclosure Gazette No. 1987-269697). 2 X 10⁵ /ml and 60 g of CYTODEX-1 (available from Pharmacia) were dispersed in DMEM culture medium 151 containing 10 % bovine serum under a gentle stirring overnight for cell adhesion onto microcarriers. From the next day the cell culture had been progressed continuously for a month with the valve SV1 adjusted to be opened for 3 seconds and closed for 10 seconds under the operating conditions: stirring within the gas/liquid mixing chamber of 300 r.p.m., aeration with pure oxygen of 3 to 4l /min., and internal pressure of the culture tank during closure of the valve SV1 of 1.2 to 1.4 kg/cm². A quantity of the culture broth was continuously collected through the culture broth collector conduit shown in Fig. 3 and the EPO activity of the culture broth thus collected was determined by the enzyme immunoassay (EIA) for EPO measurement. Fig. 6 is a graphic diagram showing the result of this measurement in the form of relative activity with respect to the activity on the culture starting day and indicates that the EPO arbitany activity can be maintained for a long period. Thus it was confirmed that the EPO had been continuously produced for a month at relatively high concentration.

During the period of this cell culture, the problem of foaming which has inconveniently accompanies the conventional suspension culture was not observed and it was demonstrated that the apparatus of the invention exhibits a high defoaming effect. The rate of oxygen supply into the culture broth was determined to be 40 mmol/h higher than the supply rates which were attained by the oxygen supply methods of prior art.

As will be apparent from the foregoing description, the present invention provides the improved arrangement such that a part of the culture tank is isolated by the filter adapted to be permeable for the culture broth as well as protein produced by the cells being cultivated but impermeable for the cell adhesion carriers to define the gas/liquid mixing chamber or zone within which oxygen can be dissolved in the culture broth at a high concentration while the desirable defoaming function is maintained. It should be understood, however, that the present invention is not limited to the embodiments as have been described above.

## Claims

1. A cell culture tank comprising a zone defined as a part of the culture tank, said zone being isolated from the remaining part by a filter adapted to be permeable for culture broth as well as protein produced by cells being cultivated but impermeable for cells or cell adhesion carriers, a conduit provided in a bottom of said zone to supply said zone with oxygen or the like, a stirring vane of high r.p.m. provided immediately above an injection nozzle associated with said conduit, 30 to 50 % of said filter's height being exposed to a gas phase area within the cell culture tank and a conduit/nozzle assembly provided immediately below a fluid level within said zone for collection of the culture broth.

2. A cell culture tank as recited in Claim 1, further comprising a defoaming vane of a high r.p.m. provided above the fluid level within the zone isolated by the filter adapted to be impermeable for the cells or the cell adhesion carriers.

3. A cell culture tank as recited in Claim 2, further comprising windows provided adjacent a top of the zone isolated by the filter adapted to be impermeable for the cells or the cell adhesion carriers.

4. A cell culture tank as recited in Claim 1, further comprising a stirring vane provided externally of the zone isolated by the filter adapted to be impermeable for the cells or the cell adhesion carriers.

5. A cell culture tank as recited in Claim 1, further comprising a germ-free pressurized vent conduit opening into the gas phase area within the cell culture tank so that the fluid level within said zone having been lowered due to foam generated within said zone isolated by the filter adapted to be impermeable for the cells or the cell adhesion carriers can be raised, said foam within said zone can be expelled out through the filter into the cell culture tank as germ-free air is supplied through said germ-free pressurized vent conduit and thus a desirable defoaming effect can be obtained.

6. A cell culture tank as recited in Claim 5, further comprising an on-off valve associated with the germ-free air vent conduit.

7. A cell culture tank as recited in Claim 1, further comprising an exhaust conduit opening into the gas phase area within the cell culture tank.

8. A cell culture tank as recited in Claim 7, further comprising on-off valves associated with said exhaust conduit opening into the gas phase area within the cell culture tank and said conduit provided in the bottom of the zone isolated by the filter adapted to be impermeable for the cells and the cell adhesion carriers, respectively.

9. A cell culture tank as recited in Claim 8, further comprising a germ-free air vent conduit opening into the gas phase area within the cell culture tank and an on-off valve associated therewith so that the fluid level within the zone isolated by the filter adapted to be impermeable for the cells or the cell adhesion carriers and having been lowered due to foam generated within said zone can be raised, said foam within said zone can be expelled out through the filter into the cell culture tank as germ-free air is supplied through said germ-free pressurized vent conduit and thus a desirable defoaming effect can be obtained.
